# EUROPÄISCHE PATENTANMELDUNG

(11) **EP 3 363 436 A1**
(43) Veröffentlichungstag der Anmeldung: **22.08.2018**
(21) Anmeldenummer: 17156568.2
(22) Anmeldetag: 16.02.2017
(51) Int. Cl.: A61K 31/4422, A61K 31/451, A61P 33/10, A61P 33/12

(54) **MITTEL ZUR BEHANDLUNG VON PARASITÄREN WURMERKRANKUNGEN**

(71) Anmelder: Haas, Helmut, 23867 Sülfeld (DE)
(72) Erfinder: Haas, Helmut, 23867 Sülfeld (DE)
(74) Vertreter: Uexküll & Stolberg

(57) **Zusammenfassung**

Die vorliegende Erfindung betrifft pharmazeutische Zusammensetzungen für die Behandlung von Wurmerkrankungen, die den Wirkstoff Felodipin oder ein pharmazeutisch geeignetes Salz oder Solvat desselben umfassen. Insbesondere betrifft die Erfindung pharmazeutische Zusammensetzungen, die als ersten Bestandteil Felodipin oder ein pharmazeutisch geeignetes Salz oder Solvat desselben und als zweiten Bestandteil Loperamid oder ein pharmazeutisch geeignetes Salz oder Solvat desselben umfassen. Die Erfindung stellt ferner Kits bereit, die diese Verbindungen umfassen.

## Beschreibung

Die vorliegende Erfindung betrifft pharmazeutische Zusammensetzungen für die Behandlung von Wurmerkrankungen, die den Wirkstoff Felodipin oder ein pharmazeutisch geeignetes Salz oder Solvat desselben umfassen. Insbesondere betrifft die Erfindung pharmazeutische Zusammensetzungen, die als ersten Bestandteil Felodipin oder ein pharmazeutisch geeignetes Salz oder Solvat desselben und als zweiten Bestandteil Loperamid oder ein pharmazeutisch geeignetes Salz oder Solvat desselben umfassen. Die Erfindung stellt ferner Kits bereit, die diese Verbindungen umfassen.

### HINTERGRUND DER ERFINDUNG

Wurmerkrankungen stellen in Entwicklungsländern und in der Veterinärmedizin ein ernstes Problem dar. Die Anzahl verfügbarer Wirkstoffe ist gering und es mehren sich Berichte über Resistenzen. Insbesondere an Infektionen mit Schistosomen sterben jährlich zahlreiche Menschen.

Schistosomen sind parasitäre Saugwürmer, die in den Blutgefäßen von Darm und Blase leben. Sie besitzen zwei Saugnäpfe, mit denen sie sich an der Gefäßinnenwand festhalten und fortbewegen. Sie ernähren sich von dem Blut des Wirts, das sie umströmt. Erwachsene Schistosomen leben in Paaren zusammen. Dabei trägt das kräftige Männchen das schlanke Weibchen in einer Bauchfalte (Pärchenegel). Schistosomen-Paare können Jahre oder sogar Jahrzehnte im Wirt überdauern. Jedes Paar produziert etwa 300 Eier täglich, die zu schwerwiegenden gesundheitlichen Schäden beim Wirt führen. Diese Schäden umfassen u.a. Entzündungen in Darm, Blase und Leber mit narbigen Veränderungen. Durch Schrumpfung der Narben in der Leber kommt es zu einer Einengung des Blutstrombetts. Diese führt in schweren Fällen zu einer Pfortaderstauung mit Aszites und Umgehungskreisläufen (Ösophagusvarizen) mit lebensbedrohlichen Blutungen.

Der Lebenszyklus von Schistosomen ist durch einen Wirtswechsel zwischen Säugetier und Wasserschnecke gekennzeichnet. Aus den mit Stuhl oder Urin des Säugetier-Endwirts ausgeschiedenen Eiern schlüpfen Flimmerlarven (Mirazidien), welche bestimmte Wasserschnecken als Zwischenwirte befallen, sich dort ungeschlechtlich vermehren und zu Gabelschwanzlarven (Zerkarien) entwickeln. Diese verlassen die Schnecken und schwärmen ins Wasser aus, um erneut ihren Säugetier-Endwirt, z.B. den Menschen, zu befallen.

Allein in Afrika sterben jährlich etwa 200.000 Menschen an den Folgen von Infektionen mit Schistosomen. 2015 wurden ca. 218 Millionen Menschen präventiv gegen Schistosomiasis behandelt, meist in subtropischen oder tropischen Ländern (WHO Schistosomiasis Fact Sheet Nr. 115, Version 2013)Die Infektion wird durch Kontakt mit Wurm-verseuchtem Wasser übertragen. Dabei sind Menschen mit regelmäßigem intensivem Wasserkontakt besonders betroffen. Die höchsten Infektionsraten findet man bei Autowäschern, Fischern und Reis-Bauern. Ebenfalls regelmäßig betroffen sind Kinder beim Baden und Frauen beim Wäschewaschen an verseuchten Wasserstellen.

Schistosomiasis wird regelmäßig mit Praziquantel behandelt. Andere Mittel sind entweder deutlich teurer und oft weniger effektiv. Die Behandlung mit Praziquantel hat jedoch auch Nachteile. So ist die Schädigung insbesondere der Jugendformen des Parasiten reversibel, d.h., einige Würmer erholen sich, sodass die Infektion nicht komplett eliminiert wird und betroffene Personen folglich weiterhin infektiöse Wurmeier ausscheiden. Praziquantel benötigt ferner zur vollen Wirkungsentfaltung die Mithilfe des Immunsystems; es wirkt nur unzureichend bei Kleinkindern mit unreifem Immunsystem und Personen mit Immundefekten. Außerdem werden in Folge der Massenbehandlung mit Praziquantel zunehmend Resistenzen gegen dieses Medikament berichtet.

Vor diesem Hintergrund liegt der vorliegenden Erfindung somit die Aufgabe zugrunde, neue wirksame und bezahlbare pharmazeutische Zusammensetzungen und Präparate bereitzustellen, mit deren Hilfe eine effektive Behandlung von Wurmerkrankungen, insbesondere von durch Schistosomen hervorgerufenen Erkrankungen, möglich ist. Erfindungsgemäß wird diese Aufgabe durch die pharmazeutischen Zusammensetzungen und Präparate gemäß den anliegenden Ansprüchen gelöst.

Im Rahmen der vorliegenden Erfindung wurde nunmehr überraschend herausgefunden, dass der Wirkstoff Felodipin bei der Behandlung von Wurmerkrankungen eingesetzt werden kann. Wie in den Beispielen gezeigt wird, führte die Inkubation von parasitären Würmern mit Felodipin zu erheblichen morphologischen Veränderungen und irreversiblen Schädigung der Würmer. Die vorliegende Erfindung stellt demgemäß in einem ersten Aspekt eine pharmazeutische Zusammensetzung für die Behandlung von Wurmerkrankungen bereit, wobei die pharmazeutische Zusammensetzung Felodipin oder ein pharmazeutisch geeignetes Salz oder Solvat desselben umfasst.

Der Wirkstoff Felodipin (Ethyl-methyl-[(4RS)-4-(2,3-dichlorphenyl)-2,6-dimethyl-1,4-dihydropyridin-3,5-dicarboxylat) gehört zur Gruppe der Calcium-Antagonisten vom 1,4-Dihydropyridin-Typ. Der Wirkstoff findet Verwendung bei der Behandlung der arteriellen Hypertonie. Felodipin wurde 2016 weltweit unter verschiedenen Markennamen (u.a. Felobeta, Felocor, Modip, Munobal, Plendil, Feloday) als Einzelpräparat vertrieben. Zusätzlich wird eine Reihe von Kombinationspräparaten von Felodipin mit anderen Blutdrucksenkern und β-Blockern vermarktet. Felodipin wird üblicherweise als Racemat aus den beiden Atropisomeren eingesetzt. Die Verwendung eines einzelnen Atropisomers ist jedoch ausdrücklich von der Erfindung umfasst. Die beiden Atropisomere weisen die folgende Strukturformel auf:

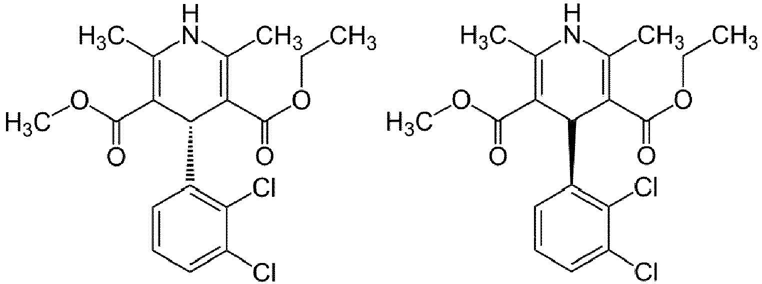

Für den Fachmann wird ersichtlich sein, dass die oben dargestellte Struktur an einer oder an mehreren Stellen substituiert oder anderweitig modifiziert werden kann, solange diese Veränderungen die anthelminthische Wirkung von Felodipin nicht wesentlich beeinträchtigen und auch unter toxischen Gesichtspunkten nicht zu nachteiligen Ergebnissen führen. Beispielsweise können eine oder mehrere Wasserstoffatome der C-H Bindungen des heterocyclischen Ringsystems durch Halogenatome, wie beispielsweise Chlor-, Brom- oder Iod-Atome, substituiert sein. Ferner kann der Wasserstoff der C-H Bindungen auch gegen eine Alkylgruppe, wie beispielsweise Methyl, Ethyl oder Propyl ersetzt werden. Ein derartig modifiziertes Felodipin ist vorliegend vom Begriff "Felodipin" mit umfasst.

In einer besonders bevorzugten Ausführungsform umfasst die pharmazeutische Zusammensetzung ferner Loperamid oder ein pharmazeutisch geeignetes Salz oder Solvat desselben. Wie in den nachstehenden Beispielen gezeigt wird, verstärkt Loperamid die Wirkung von Felodipin signifikant. Loperamid gehört zur Gruppe der Peristaltikhemmer und wird daher weltweit verbreitet für die Behandlung von Durchfallerkrankungen eingesetzt. Loperamid befindet sich auf der Liste der unentbehrlichen Arzneimittel der Weltgesundheitsorganisation (WHO). Die IUPAC Bezeichnung für Loperamid lautet 4-[4-(4-Chlorphenyl)-4-hydroxypiperidin-1-yl]-N,N-dimethyl-2,2-diphenylbutyramid.
Loperamid weist die nachfolgende Strukturformel auf:

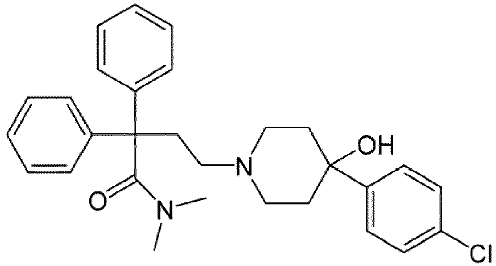

Erfindungsgemäß kann das Loperamid an einer oder an mehreren Stellen substituiert oder anderweitig modifiziert sein, solange diese Veränderungen die anthelminthische Wirkung der Substanz, die allein oder insbesondere auch in Kombination mit Felodipin beobachtet wird, nicht wesentlich beeinträchtigen und auch keine für den Patienten nicht tolerierbare toxische Nebenwirkungen nach sich ziehen. Beispielsweise können eine oder mehrere Wasserstoffatome der C-H Bindungen des heterocyclischen Ringsystems mit einem Halogenatom, wie beispielsweise einem Chlor-, Brom- oder Iod-Atom, substituiert sein. Ferner kann der Wasserstoff dieser C-H Bindungen oder der N-H-Bindungen auch gegen eine Alkylgruppe, wie beispielsweise Methyl, Ethyl oder Propyl substituiert sein. Ein derartig modifiziertes Loperamid ist vorliegend vom Begriff "Loperamid" mit umfasst.

Der Ausdruck "pharmazeutisch geeignetes Salz" bezeichnet nicht-toxische, Säure-Additionssalze sowie Alkalimetall- bzw. Erdalkalimetallsalze. Beispielhafte Säure-Additionssalze umfassen Hydrochlorid, Hydrobromid, Sulfat, Bisulfat, Acetat, Oxalat, Phosphat, Citrat, Maleat, Fumarat, Succinat, Tartrat und Laurylsulfat. Beispielhafte Alkalimetall- bzw. Erdalkalimetallsalze umfassen Natrium-, Kalium-, Calcium- und Magnesium-Salze. Darüber hinaus können erfindungsgemäß auch Ammoniumsalze und Salze mit organischen Aminen verwendet werden. Neben dem Kalziumsalz des Felodipin kann auch jedes andere pharmazeutisch geeignete kationische Salz des Felodipin eingesetzt werden. Diese Salze sind beispielsweise solche, die durch Reaktion der freien Säureform des Felodipin mit einer geeigneten Base, wie beispielsweise Natriumhydroxid, Natriummethoxid, Natriumethoxid, Natriumhydrid, Kaliummethoxid, Magnesiumhydroxid, Calciumhydroxid, Cholin, Diethanolamin und weiteren, die im Stand der Technik bekannt sind, erhalten werden.

Solvate der oben genannten Verbindungen sind ebenfalls Teil der vorliegenden Erfindung. Solvate entstehen durch die Anlagerung von einem oder mehreren Lösungsmittelmolekülen an eine erfindungsgemäß vorgeschlagene Wirkstoffverbindung (d.h. an Felodipin oder Loperamid). Ist es das Lösungsmittel Wasser, so handelt es sich um eine Hydratation. Solvate der vorgeschlagenen Wirkstoffverbindung können durch Ionenbindungen und/oder kovalente Bindungen zusammengehalten werden.

In den nachstehenden Ausführungen, die z.B. die Formulierung und Verabreichungsformen der Zusammensetzungen und Präparate betreffen, wird der Einfachheit halber oftmals lediglich auf Felodipin bzw. Loperamid verwiesen. Dieser Verweis sollte dahingehend verstanden werden, dass er einen entsprechenden Verweis auf Salze, Solvate und Derivate der Verbindungen mit einschließt.

In einem zweiten Aspekt betrifft die Erfindung ein pharmazeutisches Kombinationspräparat, das Folgendes umfasst:
a) Felodipin oder ein pharmazeutisch geeignetes Salz oder Solvat desselben, und
b) Loperamid oder ein pharmazeutisch geeignetes Salz oder Solvat desselben.

Es ist allerdings für die Ausführung der vorliegenden Erfindung nicht zwingend notwendig, dass die oben aufgeführten Komponenten in einer einheitlichen pharmazeutischen Zusammensetzung vorliegen. Vielmehr können Felodipin und Loperamid auch in getrennten Formulierungen vorliegen, die gleichzeitig oder zeitlich versetzt den zu behandelnden Patienten verabreicht werden.

Somit betrifft die Erfindung in einem zweiten Aspekt ein pharmazeutisches Kombinationspräparat, das Folgendes umfasst:
a) Felodipin oder ein pharmazeutisch geeignetes Salz oder Solvat desselben, und
b) Loperamid oder ein pharmazeutisch geeignetes Salz oder Solvat desselben,
für die gleichzeitige oder zeitlich getrennte Anwendung in der Therapie.

Dies bedeutet, dass das Felodipin gemeinsam mit dem Loperamid als einheitliche Formulierung, d.h. als einheitliche pharmazeutische Zusammensetzung, verabreicht werden kann. Die beiden Wirkstoffe der erfindungsgemäßen Kombination können beispielsweise in vitro, d.h. vor der Verabreichung an den Patienten, miteinander zu einer einheitlichen wie oben beschriebenen Darreichungsform kombiniert werden, solange keiner der beiden Bestandteile durch das Vermischen mit dem jeweils anderen Bestandteil der Kombination einen Verlust bezüglich der anthelminthischen Aktivität erleidet. So lassen sich beispielsweise Felodipin und Loperamid, die in einer lyophilisierten Mischung vorliegen, durch Zugabe eines geeigneten Lösungsmittels zu einer Lösung für die Infusion oder Injektion rekonstituieren, die beide Wirkstoffe umfasst. Darüber hinaus kann die pharmazeutische Zusammensetzung direkt als einheitliche Dosierungsform bereitgestellt werden, z.B. in Form einer Tablette für die orale Verabreichung. Gemäß einer Ausführungsform der vorliegenden Erfindung handelt es sich bei der pharmazeutischen Zusammensetzung um eine einheitliche Zusammensetzung, bei der das Felodipin und Loperamid oder deren Salze in einer einheitlichen Darreichungsform verabreicht werden.

Alternativ dazu können Felodipin und Loperamid in getrennten Formulierungen vorliegen, die zum selben Zeitpunkt oder zu unterschiedlichen Zeitpunkten an den Patienten verabreicht werden. So können beispielsweise das Felodipin und Loperamid an verschiedenen Tagen eines Behandlungszyklus verabreicht werden. Das Felodipin kann beispielsweise im Rahmen eines sich wiederholenden einwöchigen Behandlungszyklus täglich verabreicht werden, wobei Loperamid lediglich an einem bestimmten Tag dieses Zyklus verabreicht wird. Sofern das Felodipin und Loperamid zeitlich getrennt voneinander verabreicht werden sollen, ist es zweckmäßig, die Wirkstoffe in getrennten Verpackungseinheiten (z.B. in mehreren Ampullen) bereitzustellen. Dabei können Felodipin und Loperamid in gleichartigen oder unterschiedlichen Darreichungsformen vorliegen, die nachstehend genauer beschrieben werden.

Die erfindungsgemäßen pharmazeutischen Zusammensetzungen und Präparate können mit anderen anthelminthischen Mitteln kombiniert oder in Verbindung mit diesen verwendet werden. Die Zusammensetzungen und Präparate können beispielsweise gemeinsam mit herkömmlichen, im Stand der Technik bekannten Anthelminthika verabreicht werden, beispielsweise zwischen zwei aufeinanderfolgenden Zyklen einer anthelminthischen Therapie. Geeignete anthelminthische Mittel, die im Rahmen der vorliegenden Erfindung gemeinsam mit den erfindungsgemäßen Zusammensetzungen und Präparaten angewendet werden können, umfassen die gegenwärtig im Stand der Technik verfügbaren Wirkstoffe, wie z.B. Mebendazol, Praziquantel, Pyrantel, Pyrvinium oder Ivermectin. Diese Wirkstoffe werden unter den Handelsnamen Biltricide, Cesol Cysticide, Stromectol, Helmex, Molevac oder Pyrcon vertrieben.

Die pharmazeutischen Zusammensetzungen und Präparate werden gegen Erkrankungen und Infektionen eingesetzt, die von parasitären Würmern hervorgerufen werden (Helminthiasis). Die parasitären Würmern können aus verschiedenen taxonomischen Familien stammen und z.B. den Echinostomatidae, Fasciolidae, Opisthorchoiidae, Heterophyidae, Taeniidae, Hymenolepidae, Ancylostomatidae, Oxyuridae, Ascarididae, Anisakidae, Filariidae, Onchocercidae oder Hirudinea zuzuordnen sein. Bevorzugte Gattungen umfassen beispielsweise *Schistosoma, Paragonimus, Pentastomida, Trichobilharzia, Dicrocoelium, Gastrodiscoides, Watsonius, Fischoederius, Stronyloides, Dracunculus, Trichuris, Dioctophyme, Capillaria, Fasciola, Moniezia, Haemonchus, Teladorsagia, Nematodirus, Chabertia, Strongyloides, Diptocaulus, Aelurostrongylus, Crenosoma, Dictyocaulus, Rhabdias, Opisthorchis, Metorchis, Eurytrema und Philophthalmus.*

Bevorzugte Spezies umfassen beispielsweise *Trichobilharzia szidati, Dicrocoelium dendriticum, Gastrodiscoides hominis, Watsonius watsoni, Fischoederius elongatus, Stronyloides stercoralis, Dracunculus medinensis, Trichuris trichiura, Dioctophyme renale, Capillaria philippinensis, Fasciola hepatica, Moniezia expansa, Moniezia benedenii, Haemonchus contortus, Teladorsagia circumcincta, Strongyloides papillosus, Aelurostrongylus abstrusus, Angiostrongylus cantonensis, Crenosoma mephitidis, Crenosoma striatum, Crenosoma vulpis, Dictyocaulus viviparus, Paragonimus westermani, Rhabdias bufonis, Fasciola gigantica, Opisthorchis felineus, Opisthorchis viverrini, Dicrocoelium hospes, Metorchis conjunctus, Eurytrema pancreaticum, Eurytrema coelomaticum, Eurytrema ovis, Paragonimus heterotremus, Paragonimus kellicoti, Paragonimus mexicana, Paragonimus skrjabin, Paragonimus miyazakii, Paragonimus compactus, Philophthalmus lacrimosus, Philophthalmus palpebrarum, Philophthalmus gralli, Alaria americana, Onchocerca volvulus und Clinostomum complanatum.*

Die zu behandelnde Wurmerkrankung ist vorzugsweise durch Plattwürmer (Plathelminthes) oder durch Fadenwürmer (Nematoda) verursacht worden. Vorzugsweise wird die Wurmerkrankung durch Plattwürmer hervorgerufen, insbesondere durch Bandwürmer (Cestoda) oder Saugwürmer (Trematoda). Bei den Bandwürmern kann es sich um Hundebandwürmer (*Echinococcus granulosus*), Schweinebandwürmer (*Taenia solium*), Fuchsbandwürmer (*Echinococcus multilocularis*) oder Rinderbandwürmer (*Taenia saginata*) handeln. Die zu behandelnde Wurmerkrankung kann ferner durch Saugwürmer verursacht worden sein, insbesondere durch solche der Gattung *Schistosoma,* wie z.B. *Schistosoma mansoni, Schistosoma haematobium, Schistosoma intercalatum, Schistosoma japonicum, Schistosoma mekongi oder Schisostoma bovis.* Die zu behandelnde Wurmerkrankung kann darüber hinaus auch durch Fadenwürmer verursacht worden sein. Vorzugsweise wird die Wurmerkrankung durch Spulwürmer *(Ascaris lumbricoides)*, Madenwürmer (*Enterobius vermicularis)* oder Peitschenwürmer (*Trichuris trichuria)* verursacht.

Die vorliegend vorgeschlagenen pharmazeutischen Zusammensetzungen und Präparate eignen sich für die humanmedizinische sowie auch für die veterinärmedizinische Anwendung. Demgemäß kann es sich bei dem Subjekt, das mit den Zusammensetzungen und Präparaten behandelt werden soll, um einen Menschen handeln. Parasitäre Würmer kommen jedoch auch in anderen Säugetieren vor und verursachen insbesondere in Nutztierbeständen teilweise enorme wirtschaftliche Schäden. Demgemäß kann es sich bei dem zu behandelnden Subjekt ferner auch um ein Pferd, ein Rind, ein Schaf, ein Schwein, einen Hund, eine Katze oder ähnliches handeln.

Die erfindungsgemäßen Zusammensetzungen oder Präparate können in jeder im Stand der Technik bekannten, geeigneten Darreichungsform verabreicht werden. Die Zusammensetzungen und die Komponenten der Präparate können für verschiedene Verabreichungsarten formuliert sein, beispielsweise für die perorale, bukkale, sublinguale, transmukosale, rektale, subkutane intrathekale, intravenöse, intramuskuläre, intraperitoneale, nasale, intraokulare oder intraventrikuläre Verabreichung. Die erfindungsgemäßen Zusammensetzungen und die Komponenten der Präparate können darüber hinaus auch als Retardmittel formuliert werden. Demgemäß können die erfindungsgemäßen Zusammensetzungen oder die einzelnen Komponenten der erfindungsgemäßen Kombinationspräparate beispielsweise in Form von Granulaten, Pulvern, Tabletten, Kapseln, Sirup, Suppositorien, Emulsionen, Suspensionen oder Lösungen vorliegen. Entsprechende Darreichungsformen für die erfindungsgemäßen Zusammensetzungen und Präparate können mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Es ist bevorzugt, dass die erfindungsgemäßen Zusammensetzungen oder Präparate für die perorale Verabreichung formuliert sind, insbesondere Tablette, Kapsel, Granulat, Pulver oder Sirup.

Die am besten für die jeweilige Therapie geeigneten Verabreichungsarten und die zu verabreichenden Mengen von Felodipin und Loperamid können vom Fachmann problemlos mittels Routineverfahren ausfindig gemacht werden. Faktoren, die die jeweilige Menge von Felodipin und Loperamid in der zu verabreichenden pharmazeutischen Zusammensetzung beeinflussen, umfassen die Art und Intensität der Wurmerkrankung, das Alter, Körpergewicht, Geschlecht und den allgemeinen Gesundheitszustand des zu behandelnden Patienten, die gleichzeitige Verabreichung anderer therapeutischer Mittel und andere Faktoren.

Geeignete pharmazeutische Zusammensetzungen oder Präparate zur humanen Verabreichung werden in der Regel in einer Dosis von 1 mg bis 25 mg Felodipin täglich an den Patienten verabreicht werden. Vorzugsweise liegt die Menge des Felodipin im Bereich von 2 mg bis 15 mg täglich und noch stärker bevorzugt im Bereich von 5 mg täglich. Die Plasmakonzentrationen von Felodipin im Patienten liegen in der Regel zwischen 1-50 nM/L, vorzugsweise zwischen 1-25 nM/L, z.B. zwischen 1-10 nM/L. Sofern die erfindungsgemäßen pharmazeutischen Zusammensetzungen oder Präparate Felodipin in Kombination mit Loperamid umfassen, so werden diese so formuliert sein, dass die tägliche Dosis an Loperamid zwischen 1-20 mg liegt, vorzugsweise zwischen 1-15 mg, zwischen 1-10 mg, wie z.B. 2 mg. Die Plasmakonzentrationen von Loperamid im Patienten liegen in der Regel zwischen 1-25 nM/L, vorzugsweise zwischen 1-10 nM/L, z.B. zwischen 1-5 nM/L.

Die therapeutische Wirksamkeit der erfindungsgemäßen pharmazeutischen Zusammensetzungen und Präparate kann anhand von im Stand der Technik bekannten Methoden und Parametern bewertet werden. Diese Parameter umfassen u.a. die Wirksamkeit der erfindungsgemäßen Zusammensetzung bei der Beseitigung der parasitären Würmer, z.B. aus dem Darm des Patienten, Zählung der Wurmeier im Stuhl, serologische Tests auf Parasitenantigene im Blut des Patienten, Verlaufskontrollen Wurm-spezifischer Immunglobulin-Konzentrationen, Serum-IgE, Blutbilduntersuchungen (z.B. Rückgang der Eosinophilie), Zeit bis zum Fortschreiten der Erkrankung und die Überlebensrate der behandelten Patienten. Eine anthelminthische Wirkung kann sich durch eine Inhibierung des Wurmwachstums und/oder der Wurmvermehrung, durch eine Verzögerung des Wurmwachstums und/oder der Wurmvermehrung, durch eine Verkleinerung der von Würmern befallenen Körperareale, durch eine Verringerung der Anzahl von Würmern, durch eine Verlängerung der Zeitspanne bis zum Einsetzen eines erneuten Wurmwachstums und/oder einer Wurmvermehrung oder durch eine Verzögerung im Fortschreiten der Erkrankung darstellen.

Vorzugsweise führen die erfindungsgemäßen Zusammensetzungen und Präparate zu einem vollständigen Ansprechen beim Patienten. Mit vollständigem Ansprechen sind die Beseitigung sämtlicher klinisch nachweisbaren Erkrankungssymptome sowie die Wiederherstellung von normalen Befunden im Blutbild, bei sonographischen Untersuchungen, Röntgenuntersuchungen, CT-Aufnahmen und ähnlichem gemeint. Ein solches Ansprechen dauert mindestens einen Monat nach Absetzen der erfindungsgemäßen Behandlung an. Die anthelminthisch wirksamen Zusammensetzungen und Präparate der vorliegenden Erfindung können zu einem partiellen Ansprechen beim Patienten führen. Bei einem partiellen Ansprechen kommt es zu einer Verringerung der messbaren Parasitenbelastung im Patienten. Dies bedeutet insbesondere, dass die Anzahl der im Patienten vorhandenen Parasiten abnimmt und keine neuen befallenen Körperareale zu beobachten sind. Gleichzeitig kommt es zu einer Verbesserung eines oder mehrerer durch die Krankheit verursachter Symptome (z.B. Fieber, Gewichtsverlust, abdominale Beschwerden, Anämie, Hinfälligkeit).

Die Erfindung betrifft in einem weiteren Aspekt ein Verfahren zur Behandlung eines Patienten, der an einer Wurmerkrankung leidet, bei dem man dem Patienten Felodipin oder ein pharmazeutisch geeignetes Salz oder Solvat desselben verabreicht. In einer bevorzugten Ausführungsform der vorliegenden Erfindung wird das Felodipin in Kombination mit Loperamid oder ein pharmazeutisch geeignetes Salz oder Solvat desselben verabreicht. Die Verabreichung von Felodipin kann gleichzeitig oder zeitlich getrennt von der Verabreichung von Loperamid erfolgen. Dies bedeutet, dass die Kombination aus Felodipin und Loperamid wie oben beschrieben entweder in einer einheitlichen Darreichungsform (d.h. in einer einzigen pharmazeutischen Zusammensetzung) vorliegen kann, die an den Patienten verabreicht wird, oder in getrennten Darreichungsformen, die zu verschiedenen Zeitpunkten, d.h. zeitlich getrennt voneinander, an den Patienten verabreicht werden.

In einer bevorzugten Ausführungsform der vorliegenden Erfindung wird Felodipin vor Verabreichung von Loperamid an den Patienten verabreicht. Vorzugsweise wird die Verabreichung von Felodipin 1-24 h vor Verabreichung von Loperamid durchgeführt. In einer besonders bevorzugten Ausführungsform wird die Verabreichung von Felodipin 12-16 h vor Verabreichung von Loperamid durchgeführt. In einer alternativen Ausführungsform der vorliegenden Erfindung wird Loperamid vor Verabreichung von Felodipin an den Patienten verabreicht. Vorzugsweise wird die Verabreichung von Loperamid 1-24 h vor Verabreichung von Felodipin durchgeführt. In einer besonders bevorzugten Ausführungsform wird die Verabreichung von Loperamid 12-16 h vor Verabreichung von Felodipin durchgeführt.

In einem dritten Aspekt betrifft die Erfindung einen Kit, der die folgenden Bestandteile umfasst:
a) Felodipin oder ein pharmazeutisch geeignetes Salz oder Solvat desselben,
b) Loperamid oder ein pharmazeutisch geeignetes Salz oder Solvat desselben, und
c) optional, Anweisungen zur kombinierten Verwendung von Felodipin und Loperamid bei der Behandlung eines Patienten mit einer Wurmerkrankung.

Die Anweisungen zur kombinierten Verwendung von Felodipin und Loperamid bei der Behandlung eines Patienten mit einer Wurmerkrankung beschreiben dem Anwender die Schritte, die bei der Herstellung und Verabreichung der erfindungsgemäßen pharmazeutischen Zusammensetzungen und Präparate durchzuführen sind, um zu dem gewünschten therapeutischen Ergebnis zu gelangen. Die Anweisungen können beispielsweise spezifische Mengen und Behandlungspläne umfassen, und sie können Informationen bezüglich der Verabreichungsdauer einschließen. Der Kit kann darüber hinaus andere Bestandteile umfassen, die zur Durchführung der vorliegenden Erfindung nützlich sind.

Der Kit kann beispielsweise geeignete Lösungsmittel, Hilfsstoffe, Bindemittel, Verdünnungsmittel oder ähnliche Substanzen umfassen. Ferner kann der Kit auch Injektionsspritzen, Kanülen, Katheter und andere Hilfsmittel für die Verabreichung der erfindungsgemäßen pharmazeutischen Zusammensetzungen und Präparate umfassen. Die pharmakologisch wirksamen Substanzen können in dem Kit in jeder beliebigen Form bereitgestellt werden, die ihre pharmakologische Aktivität erhält. So lassen sich Felodipin und Loperamid beispielsweise in gelöster oder lyophilisierter Form bereitstellen.

### BESCHREIBUNG DER FIGUREN

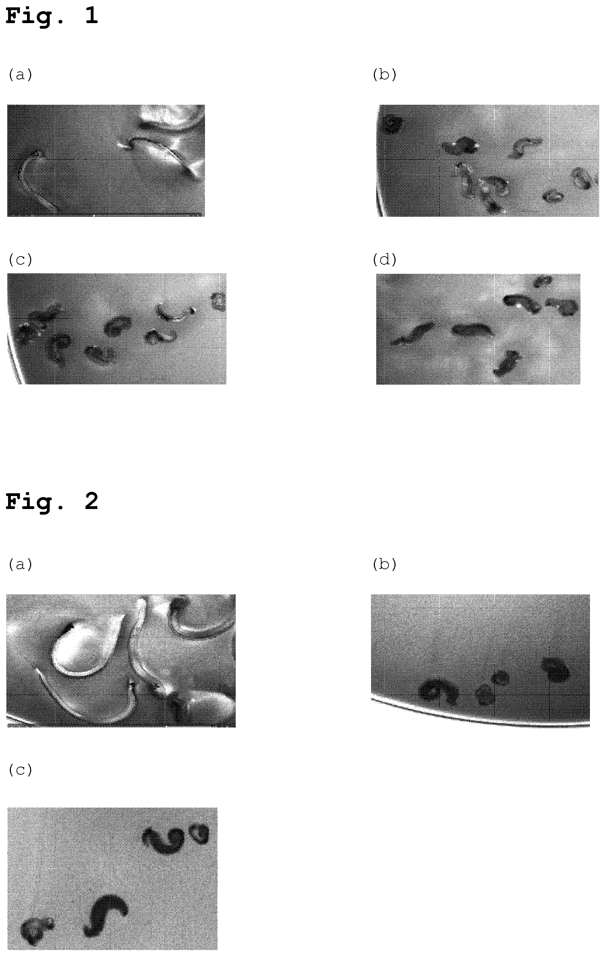

Figur 1 zeigt die Ergebnisse der Inkubation von Schistosomen mit verschiedenen Konzentrationen von Felodipin. Figur 1 (a) zeigt adulte Schistosomen vor Gabe von verschiedenen Felodipin-Konzentrationen. Figur 1 (b) zeigt die Wirkung von 10 µM Felodipin auf die Schistosomen 24 h nach Gabe von Felodipin. Figur 1 (c) zeigt die Wirkung von 20 µM Felodipin auf die Schistosomen 24 h nach Gabe von Felodipin. Figur 1 (d) zeigt die Wirkung von 40 µM Felodipin auf die Schistosomen 24 h nach Gabe von Felodipin.
Figur 2 zeigt die Ergebnisse der Inkubation von Schistosomen mit verschiedenen Konzentrationen von Felodipin 7 Tage nach Gabe von Felodipin. Figur 2 (a) zeigt Kontroll-Schistosomen am Tag 7. Figur 2 (b) zeigt die Wirkung von 20 µM Felodipin auf die Schistosomen am Tag 7 nach Gabe von Felodipin. Figur 2 (c) zeigt die Wirkung von 40 µM Felodipin auf die Schistosomen am Tag 7 nach Gabe von Felodipin.

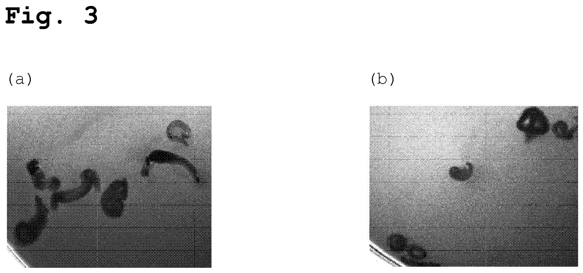

Figur 3 zeigt die Ergebnisse der Inkubation von Schistosomen mit verschiedenen Konzentrationen von Felodipin und Loperamid. Figur 2 (a) zeigt die Wirkung von 10 µM Felodipin und 10 µM Loperamid auf die Schistosomen am Tag 7 nach Gabe der Wirkstoffkombination. Figur 2 (a) zeigt die Wirkung von 20 µM Felodipin und 20 µM Loperamid auf die Schistosomen am Tag 7 nach Gabe der Wirkstoffkombination.

### BEISPIELE

### Beispiel 1: Infektion von Wasserschnecken

Mirazidien für die Infektion von Wasserschnecken (*Biomphalaria glabrata*, Stamm Puerto Rico) wurden mittels Standardverfahren aus Gewebe (Leber, Darm) von Syrischen Goldhamstern bzw. Mäusen gewonnen, die mit *S. mansoni* (Liberian strain bzw. Brasilian strain) infiziert worden waren. Hierzu wurde das Gewebe (freundlicherweise zur Verfügung gestellt von Prof. Dr. Grevelding, Universität Gießen bzw. von Dr. Sombetzki, Universität Rostock) mechanisch zerkleinert und die darin enthaltenen Wurmeier mittels Enzymverdau aus dem Gewebeverband herausgelöst. Nach Zugabe von H₂O zu dem Gewebspräzipitat schlüpfen aus den Eiern die Mirazidien. Die Infektion erfolgte separat für jede Schnecke mit 4-7 Mirazidien. Da Licht für die Zerkarien der Schlüsselreiz zum Verlassen der Schnecke ist, wurden die Schnecken in der vierten Woche nach Infektion dunkel gestellt, um ein vorzeitiges Ausschwärmen von Zerkarien zu unterbinden.

### Beispiel 2: Gewinnung von Zerkarien

6-7 Wochen nach Infektion wurden die infizierten Schnecken aus Beispiel 1 in Wasser mittels einer 60-Watt Glühlampe von oben belichtet, da ab diesem Zeitpunkt Zerkarien die infizierten Schnecken in ausreichender Menge verlassen. Während einer Belichtungsdauer von 2-3 Stunden wurden die aufschwimmenden Zerkarien gesammelt. Nach wiederholtem Waschen und Überführen in Medium erfolgte die Transformation der Zerkarien mechanisch nach Standardverfahren. Dazu erfolgten wiederholte Passagen durch eine Emulsionsnadel, an die zwei Spritzen angeschlossen sind. Die durch die Transformation aus den Zerkarien (Gabelschwanzlarven) entstandenen Schistosomula wurden ebenfalls wiederholt in Medium gewaschen und dabei von den abgescherten Schwänzen getrennt.

### Beispiel 3: Kultivierung der Schistosomula

Die transformierten Schistosomula wurden nach dem von Basch beschriebenen Verfahren kultiviert (Basch (1981), J Parasitol.; 67: 179-185), wobei das Verfahren leicht modifiziert wurde, wie es bei Reimers beschrieben wurde (Reimers et al. (2015), PLoS Negl Trop Dis 9(3): e0003593). Die Schistosomula entwickelten sich ähnlich wie unter *in vivo* Bedingungen innerhalb von drei Wochen zu juvenilen Schistosomen und erreichten nach 5-7 Wochen das Erwachsenenstadium inklusive Paarbildung. Die Ablage unreifer Eier erfolgte einige Wochen später.

### Beispiel 3: Wirkstofftestung an Schistosomen

Mehrere Substanzbibliotheken wurden auf eine Wirksamkeit gegen Schistosomen untersucht. Dazu wurden verschiedene Entwicklungsstadien von *S. mansoni* (Schistosomula, juvenile Würmer und adulte gepaarte und ungepaarte Würmer) *in vitro* mit den Einzelsubstanzen in einer Konzentration von zunächst 10 µM versetzt. Der Wirkstoff wurde dabei üblicherweise 24 h auf den Würmern belassen und dann durch sechsmaligen Wechsel von jeweils 50% des Mediums weitgehend ausgewaschen, um den *in vivo* erfolgenden Abfall der Wirkstoffkonzentration *in vitro* nachzustellen. Die jeweiligen Wirkstoff-Effekte wurden nach verschiedenen Zeitintervallen (4 h, 24 h, 48 h, 72 h und 7 Tage) beurteilt und photographisch sowie - bei positivem Ergebnis - per Videoaufnahme dokumentiert. Die Ergebnisse sind in den Figuren 1-3 dargestellt. Es lässt sich erkennen, dass sowohl Felodipin als auch eine Kombination von Felodipin und Loperamid hochwirksam Schistosomen abtöten.

## Patentansprüche

1. Pharmazeutische Zusammensetzung, die Felodipin oder ein pharmazeutisch geeignetes Salz oder Solvat desselben umfasst, zur Verwendung in einem Verfahren zur Behandlung von Wurmerkrankungen.

2. Pharmazeutische Zusammensetzung zur Verwendung in einem Verfahren gemäß Anspruch 1, **dadurch gekennzeichnet, dass** die Zusammensetzung für die perorale Verabreichung formuliert ist.

3. Pharmazeutische Zusammensetzung zur Verwendung in einem Verfahren gemäß Anspruch 1 oder 2, **dadurch gekennzeichnet, dass** die Zusammensetzung ferner Loperamid oder ein pharmazeutisch geeignetes Salz oder Solvat desselben umfasst.

4. Pharmazeutische Zusammensetzung zur Verwendung in einem Verfahren gemäß einem der Ansprüche 1 bis 3, **dadurch gekennzeichnet, dass** die Zusammensetzung ferner einen pharmazeutisch annehmbaren Träger oder Hilfsstoff umfasst.

5. Pharmazeutische Zusammensetzung zur Verwendung in einem Verfahren gemäß einem der Ansprüche 1 bis 4, **dadurch gekennzeichnet, dass** die Wurmerkrankung durch Plattwürmer (Plathelminthes) oder Fadenwürmer (Nematoda) verursacht wird.

6. Pharmazeutische Zusammensetzung zur Verwendung in einem Verfahren gemäß Anspruch 5, **dadurch gekennzeichnet, dass** es sich bei den Plattwürmern um Bandwürmer (Cestoda) oder Saugwürmer (Trematoda) handelt.

7. Pharmazeutische Zusammensetzung zur Verwendung in einem Verfahren gemäß Anspruch 6, **dadurch gekennzeichnet, dass** es sich bei den Bandwürmern um Hundebandwürmer (*Echinococcus granulosus*), Schweinebandwürmer (*Taenia solium*), Fuchsbandwürmer *multilocularis*) oder Rinderbandwurm (*Taenia saginata*) handelt.

8. Pharmazeutische Zusammensetzung zur Verwendung in einem Verfahren gemäß Anspruch 6, **dadurch gekennzeichnet, dass** es sich bei den Saugwürmern um solche der Gattung *Schistosoma* handelt.

9. Pharmazeutische Zusammensetzung zur Verwendung in einem Verfahren gemäß Anspruch 8, **dadurch gekennzeichnet, dass** es sich bei den Saugwürmern der Gattung *Schistosoma* um *Schistosoma mansoni, Schistosoma haematobium, Schistosoma intercalatum, Schistosoma japonicum, Schistosoma mekongi oder Schisostoma bovis* handelt.

10. Pharmazeutische Zusammensetzung zur Verwendung in einem Verfahren gemäß Anspruch 5, **dadurch gekennzeichnet, dass** es sich bei den Fadenwürmern um Spulwürmer *(Ascaris lumbricoides),* Madenwürmer (*Enterobius vermicularis),* Filarien (z.B. *Onchocerca volvulus)* oder Peitschenwürmer (*Trichuris trichiura)* handelt.

11. Pharmazeutisches Kombinationspräparat, umfassend:
a) Felodipin oder ein pharmazeutisch geeignetes Salz oder Solvat desselben, und
b) Loperamid oder ein pharmazeutisch geeignetes Salz oder Solvat desselben,
für die gleichzeitige oder zeitlich getrennte Anwendung in der Therapie.

12. Pharmazeutisches Kombinationspräparat gemäß Anspruch 11, **dadurch gekennzeichnet, dass** Felodipin oder das pharmazeutisch geeignete Salz oder Solvat desselben und Loperamid oder das pharmazeutisch geeignete Salz oder Solvat desselben für die getrennte Verabreichung formuliert sind.

13. Pharmazeutisches Kombinationspräparat gemäß Anspruch 12, **dadurch gekennzeichnet, dass** Felodipin oder das pharmazeutisch geeignete Salz oder Solvat desselben und Loperamid oder das pharmazeutisch geeignete Salz oder Solvat desselben in unterschiedlichen Darreichungsformen formuliert sind.

14. Pharmazeutisches Kombinationspräparat gemäß einem der Ansprüche 11-13 zur Verwendung in einem Verfahren zur Behandlung von Wurmerkrankungen.

15. Kit, umfassend
a) Felodipin oder ein pharmazeutisch geeignetes Salz oder Solvat desselben,
b) Loperamid oder ein pharmazeutisch geeignetes Salz oder Solvat desselben.
